# EUROPEAN PATENT APPLICATION

(11) **EP 4 427 749 A1**
(43) Date of publication of application: **11.09.2024**
(21) Application number: 22890359.7
(22) Date of filing: 02.11.2022
(51) Int. Cl.: A61K 31/473, A61P 1/00, A61P 29/00, A61K 9/00

(54) **USE OF APOMORPHINE AS THERAPEUTIC AGENT FOR NECROPTOSIS-RELATED DISEASES**

(30) Priority: 02.11.2021 KR 20210148543
(71) Applicant: Industry-Academic Cooperation Foundation, Yonsei University, Seoul 03722 (KR)
(72) Inventor: SHIN, Jeon-Soo, Seoul 03698 (KR); HAN, Myeonggil, Goyang-si Gyeonggi-do 10503 (KR); PARK, In Ho, Seoul 06684 (KR); KWAK, Man Sup, Seoul 02843 (KR)
(74) Representative: Vossius & Partner Patentanwälte Rechtsanwälte mbB
(86) International application number: PCT/KR2022/017018
(87) International publication number: WO 2023/080628

(57) **Abstract**

The present invention provides novel use of apomorphine for the prevention or treatment of necroptosis-related diseases. The present invention relates to a pharmaceutical composition for the prevention or treatment of necroptosis-related disease, comprising: apomorphine or a pharmaceutically acceptable salt thereof; and a pharmaceutically acceptable carrier.

## Description

### [Technical Field]

The present invention relates to apomorphine for treating a necroptosis-related disease and a use thereof, and more particularly, to a pharmaceutical composition for use in preventing or treating a necroptosis-related disease, which includes apomorphine or a pharmaceutically acceptable salt thereof as an active ingredient, and a method of preparing the same.

### [Background Art]

Proper cell death is one of the most important cellular processes in the life of living organisms, and inappropriate cell death is closely related to human diseases such as cancer, degenerative diseases, immune diseases, and abnormal development phenomena. For this reason, the phenomenon of cell death, the phenomenon of regulating cell death, and human diseases occurring by incorrect regulation and the development of treatment methods of overcoming them are well known as a sector that is most widely researched in the field of life sciences.

Generally, cell death includes necrosis and apoptosis. Apoptosis is programmed cell death regulated during development and physiological cell turnover. Necrosis is direct and rapid cell death caused by externally-induced physical and chemical stress (heat, osmotic pressure, pressure, or shock caused by temperature difference), and this necrosis results from uncontrolled and unprogrammed accidental causes and causes the loss of a cell membrane and cell collapse. In addition, as the cell membrane is lost, intracellular materials cause inflammation. However, many recent research results have revealed that necrosis is not accidental cell death, but a programmed cell death mechanism similar to apoptosis, and the term necroptosis (or programmed necrosis) was coined to explain this.

Necroptosis is a programmed cell death mode of cells. The majority of research related to cell death has focused on apoptosis, and with the discovery of the enzyme caspase, many pharmaceutical companies have been developing drugs using caspase inhibitors for the past 10 years. However, there are still very few drugs approved by the FDA, and this is because apoptosis is cell death that occurs in physiological situations and is likely to be caused by a defense mechanism that maintains homeostasis in the body.

In contrast, necroptosis is cell death that occurs mainly in pathological situations and is accompanied by an inflammatory response in most cases. Representative diseases caused by necroptosis are ischemic, neurodegenerative, and inflammatory diseases.

The above-described necroptosis is uncontrolled accidental death in pathological situations, and since its mechanism of action, molecular target, and signal transduction system have not been studied much, there is a very urgent need to discover and develop necroptosis inhibitors to treat causative diseases and identify its biological and pathological causes. However, no suitable treatments for the above types of necroptosis disease have been reported yet.

Meanwhile, apomorphine (APO) is a compound with the chemical formula C₁₇H₁₇NO₂, and is a derivative of a narcotic synthesized by reacting morphine with a strong mineral acid. It is an FDA-approved drug that is routinely used to treat Parkinson's disease through the activation of dopamine D2-like receptors. It is structurally similar to dopamine and acts by exciting dopamine autoreceptors located in dopaminergic neurons. Particularly, apomorphine activates dopamine receptors in the nigrostriatal pathway, the limbic system, the hypothalamus, and the pituitary gland, causing an effect such as improved motor function.

In the related art, apomorphine has been used to treat vomiting, erectile dysfunction, and Parkinson's disease. In addition, there are related art documents disclosing that apomorphine can be used as a treatment for myocardial infarction (see Patent Document 0001), a treatment for neurodegenerative diseases, including motor neuron disease, amyotrophic lateral sclerosis (ALS, Lou Gehrig's disease), primary lateral sclerosis (PLS), Huntington's disease, Alzheimer's disease, Parkinson's disease, and age-related macular degeneration (see Patent Document 0002), or a treatment for ischemic diseases, including ischemic cerebral apoplexy, ischemic cerebral thrombosis, ischemic cerebral embolism, hypoxic-ischemic encephalopathy, ischemic cardiac disease, ischemic enteropathy, peripheral ischemia disease, or ischemic reperfusion-induced arrhythmias (see Patent Document 0003), but none of these documents revealed the necroptosis-inhibiting action of apomorphine.

### [Related Art Documents]

### [Patent Documents]

(Patent Document 0001) CN Unexamined Patent Publication No. 105753879 A
(Patent Document 0002) US Unexamined Patent Publication No. 2021-0214310 A1
(Patent Document 0003) US Unexamined Patent Publication No. 7294715 B2

### [Disclosure]

### [Technical Problem]

The present invention is directed to providing a drug having a necroptosis-inhibiting function.

The present invention is also directed to providing a drug having therapeutic efficacy for a necroptosis-related disease.

### [Technical Solution]

To address the above problems, as a result of the present inventors performing drug screening for identifying a material with a function of inhibiting necroptosis, it was revealed that apomorphine has a necroptosis inhibitory function and efficacy in treating a necroptosis-related disease.

Therefore, the present invention provides a pharmaceutical composition for use in preventing or treating a necroptosis-related disease, which includes apomorphine or a pharmaceutically acceptable salt thereof as an active ingredient.

### [Advantageous Effects]

The present invention provides a pharmaceutical composition, which includes apomorphine as an active ingredient and can be effectively used in the prevention and treatment of a necroptosis-related disease.

### [Description of Drawings]

FIG. 1A is a graph confirming a propidium iodide (PI) uptake rate when a human monoclonal cell line, i.e., THP-1 cells, was treated with TBZ+apomorphine (APO). Data is presented as the average of two experiments (TBZ: TNF-α (ATGen, TNF0501) + BV6 (Biovision, B1332-5) + Z-VAD (invivoGen, tlrl-vad)).
FIG. 1B shows images and a graph, which confirm changes in MLKL phosphorylation through Western blotting when THP-1 cells are treated with TBZ+APO. Data is presented as the average of two independent experiments.
FIG. 1C shows images obtained by fractionating cells treated with TBZ, necrostatin-1 (NEC-1), and APO into cytoplasmic and membrane fractions, and treating samples with the indicated antibodies through Western blotting. LDH is a cytoplasmic marker, LAMP1 is a membrane marker, and data is presented as the average of two independent experiments.
FIG. 1D shows an IC50 curve for apomorphine treatment.
FIG. 2A shows images obtained by separating cells treated with each molecule for the indicated time into a cytoplasmic fraction and a membrane fraction, analyzing them through non-reducing or reducing SDS-PAGE, and then investigating them with a specific antibody. LDH is a cytoplasmic marker, and LAMP1 is a membrane marker.
FIG. 2B shows images obtained by analyzing oligomerized MLKL over time through non-reducing or reducing SDS-PAGE after inducing necroptosis.

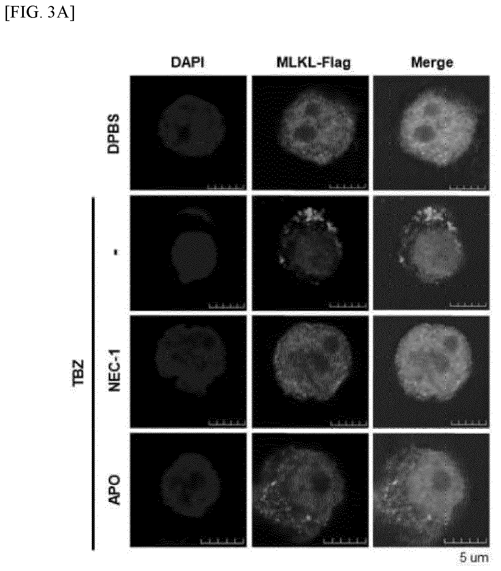

FIG. 3A shows images obtained by treating transfected THP-1 cells with TBZ or NEC-1, APO, staining them with DAPI and Alexa 488, and then observing them through confocal microscopy.

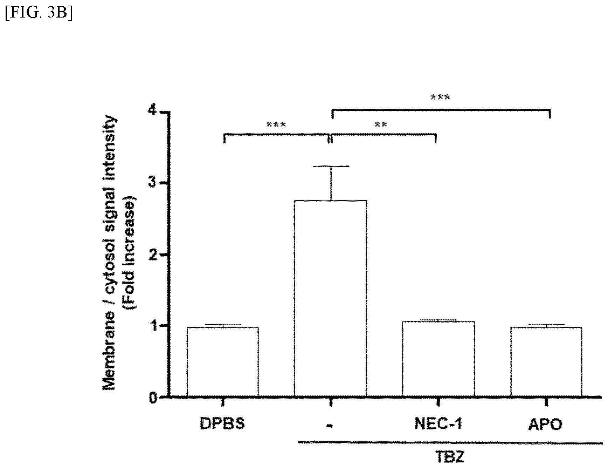

FIG. 3B shows a graph for a fold increase in membrane/cytoplasmic signal intensity of Flag-MLKL (Scale bar: 5 µm, mean ± SEM, *** p* < 0.01, *** *p* < 0.001 vs TBZ-only group, One-way ANOVA).
FIG. 4 show images and a graph, which are obtained by treating TBZ-treated THP-1 cells with APO in a concentration-dependent manner (1, 5, 20, 80, and 320 µM), concentrating a supernatant, and confirming it with an HMGB1 antibody through Western blotting. Data is presented as the average of three independent experiments (mean ± SEM, *** *p* < 0.001 vs TBZ-only group, One-way ANOVA).

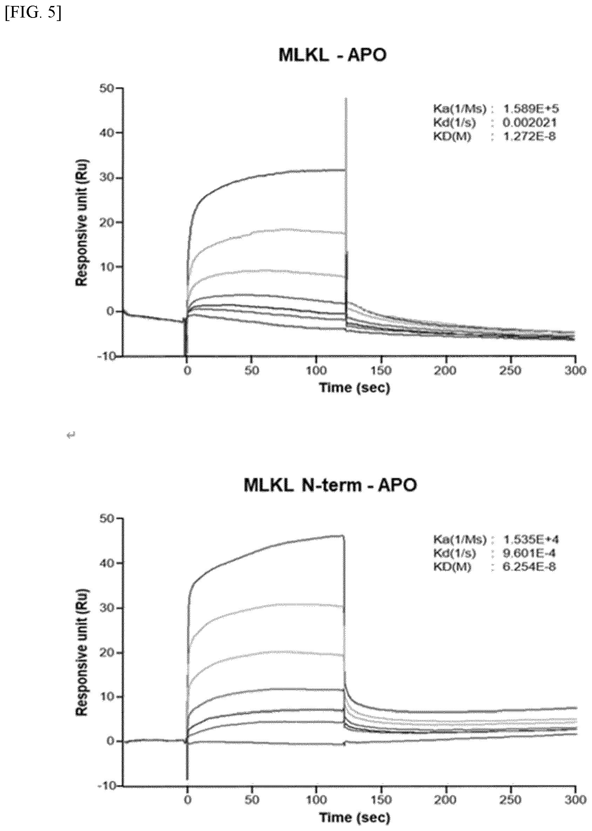

FIG. 5 shows graphs analyzing the surface plasmons of APO (200, 100, 50, 25, 12.5 6.25, and 0 µg/ml) binding to MLKL and an MLKL N-term domain.
FIG. 6A shows an image analyzing the oligomerization of MLKL according to the APO (oxidized and reduced forms) treatment of TBZ-treated THP-1 cells through non-reducing or reducing SDS-PAGE.
FIG. 6B shows graphs analyzing the surface plasmons of oxidized and reduced forms of APO (200, 100, 50, 25, 12.5, 6.25, and 0 µg/ml) binding to MLKL protein.
FIG. 6C shows graphs analyzing the surface plasmons of oxidized and reduced forms of APO (250, 125, 62.5, 31.12, 15.56, 7.88, 3.44, 1.72, 0.86, and 0 µg/ml) binding to the MLKL N-term domain.
FIG. 7A shows images and a graph, which show an effect of APO injection (0.15, 1.5, or 15 mg/kg) on histopathology and changes in mucosal substances of the mouse colon and HI scores (Scale bar: 100 µm, mean ± SD (n = 5). * *p* < 0.05, *** *p* < 0.001, One-way ANOVA).
FIG. 7B shows images and a graph, which show the colon length of each group (mean ± SD (n = 5). * *p <* 0.05, ** *p <* 0.01, *** *p <* 0.001, One-way ANOVA).
FIG. 7C shows graphs evaluating the disease activity index (DAI) of colitis. NEC-1 was used as an inhibitor control (mean ± SD (n = 5). ** p* < 0.05, *** *p* < 0.001, t-test).

### [Modes of the Invention]

The present invention relates to a pharmaceutical composition for use in preventing or treating a necroptosis-related disease, which includes apomorphine as an active ingredient.

The term "necroptosis" used herein refers to programmed cell death, programmed necrosis, and necrosis. Necroptosis leads to the release of danger-associated molecular patterns (DAMPs), and a representative DAMP in the extracellular environment is HMGB1, which triggers the activation of the immune system and causes inflammation. Necroptosis, which is programmed cell death, is achieved by sequential signaling of RIP1, RIP3, and MLKL in the cytoplasm, and in the final step, upon phosphorylation, the MLKL molecule is oligomerized in the cell membrane and pores are formed in the cell membrane. Specifically, phosphorylated MLKL (p-MLKL) forms a tetramer through oligomerization. Two tetramers interact to form an octamer, and pores are formed in a cell membrane. For further details in this regard, reference may be made to Sun L, et al. Trends Biochem Sci. 2014 Dec;39(12):587-93. doi: 10.1016/j.tibs.2014.10.003. PMID: 25455759.

In this regard, in the present invention, it was confirmed that apomorphine specifically inhibits the formation of pores in the cell membrane, mediated by mixed lineage kinase domain-like pseudokinase (MLKL), which is a terminal mediator of necroptosis.

As an oxidative reaction occurs, the direct binding of the apomorphine of the present invention with MLKL increases, and thus MLKL oligomerization is inhibited, which ultimately inhibits necroptosis.

Therefore, the present invention provides a novel use of apomorphine for preventing or treating a necroptosis-related disease.

The necroptosis-related disease of the present invention may be an ischemic, neurodegenerative, or inflammatory disease. Specifically, the necroptosis-related disease may include stroke, autoimmune diseases, inflammatory bowel disease (IBD), sepsis, chronic obstructive pulmonary disease, acute respiratory distress disorder, transfusion-related acute lung injury, transplant rejection, atherosclerosis, aortic aneurysms, myocardial infarction, terminal ileitis, acute kidney injury, renal ischemia reperfusion injury, liver injury, steatohepatitis, pancreatitis, or bone marrow failure. For related information, reference may be made to Khoury MK, Gupta K, Franco SR, Liu B. Necroptosis in the Pathophysiology of Disease. Am J Pathol. 2020 Feb;190(2):272-285. doi: 10.1016/j.ajpath.2019.10.012. Epub 2019 Nov 26. PMID: 31783008; PMCID: PMC6983729; Ware LB. Transfusion-induced lung endothelial injury: a DAMP death? Am J Respir Crit Care Med. 2014 Dec 15;190(12):1331-2. doi: 10.1164/rccm.201411-2047ED. PMID: 25496097.; Leeper NJ. The role of necroptosis in atherosclerotic disease. JACC Basic Transl Sci. 2016 Oct;1(6):548-550. doi: 10.1016/j.jacbts.2016.08.002. PMID: 28480338; PMCID: PMC5419689.; Gunther C, Martini E, Wittkopf N, Amann K, Weigmann B, Neumann H, Waldner MJ, Hedrick SM, Tenzer S, Neurath MF, Becker C. Caspase-8 regulates TNF-α-induced pithelial necroptosis and terminal ileitis. Nature. 2011 Sep 14;477(7364):335-9. doi: 10.1038/nature10400. PMID: 21921917; PMCID: PMC3373730.; Jun W, Benjanuwattra J, Chattipakorn SC, Chattipakorn N. Necroptosis in renal ischemia/reperfusion injury: A major mode of cell death? Arch Biochem Biophys. 2020 Aug 15;689:108433. doi: 10.1016/j.abb.2020.108433. Epub 2020 May 26. PMID: 32470461.; Gautheron J, Vucur M, Luedde T. Necroptosis in Nonalcoholic Steatohepatitis. Cell Mol Gastroenterol Hepatol. 2015 Mar 12;1(3):264-265. doi: 10.1016/j.jcmgh.2015.02.001. PMID: 28210679; PMCID: PMC5301189.; Roderick JE, Hermance N, Zelic M, Simmons MJ, Polykratis A, Pasparakis M, Kelliher MA. Hematopoietic RIPK1 deficiency results in bone marrow failure caused by apoptosis and RIPK3-mediated necroptosis. Proc Natl Acad Sci U S A. 2014 Oct 7;111(40):14436-41. doi: 10.1073/pnas.1409389111. Epub 2014 Sep 22. PMID: 25246544; PMCID: PMC4209989.

The inflammatory bowel disease may include Crohn's disease or ulcerative colitis. The autoimmune disease may include psoriasis or rheumatoid arthritis.

The present inventors found that apomorphine is effective in improving the symptoms of inflammatory bowel disease through the DSS-induced colitis experiment of the following examples.

Therefore, the present invention provides a pharmaceutical composition for use in preventing or treating a necroptosis-related disease, which includes apomorphine or pharmaceutically acceptable salt thereof as an active ingredient, a use of apomorphine or a pharmaceutically acceptable salt thereof for preparing the pharmaceutical composition for preventing or treating a necroptosis-related disease, and a method of preventing or treating a necroptosis-related disease, which includes administering a therapeutically effective amount of apomorphine or a pharmaceutically acceptable salt thereof to a subject.

The pharmaceutically acceptable salt of the present invention may be prepared by a conventional method in the corresponding art, and examples of the pharmaceutically acceptable salt include salts of inorganic acids such as hydrochloric acid, bromic acid, sulfuric acid, sodium bisulfate, phosphoric acid, nitric acid, and carbonic acid, salts of organic acids such as formic acid, acetic acid, propionic acid, oxalic acid, succinic acid, benzoic acid, citric acid, maleic acid, malonic acid, tartaric acid, gluconic acid, lactic acid, gentisic acid, fumaric acid, lactobionic acid, salicylic acid, trifluoroacetic acid, and acetylsalicylic acid (aspirin), salts of amino acids such as glycine, alanine, vanillin, isoleucine, serine, cysteine, cystine, aspartic acid, glutamine, lysine, arginine, tyrosine, and proline, salts of sulfonic acids such as methanesulfonic acid, ethanesulfonic acid, benzenesulfonic acid, and toluenesulfonic acid, metal salts resulting from reactions with an alkali metal such as sodium and potassium, and salts with ammonium ions. The pharmaceutically acceptable salts are not particularly limited as long as they can be used in pharmaceuticals.

In one embodiment of the present invention, apomorphine may be in the form of a salt. The pharmaceutical composition of the present invention may include, but not limited to, apomorphine hydrochloride as an active ingredient. More particularly, in the present invention, apomorphine may be apomorphine hydrochloride hemihydrate.

The pharmaceutical composition of the present invention includes one or more pharmaceutically acceptable carriers in addition to the active ingredient. As used herein, "pharmaceutically acceptable carrier" refers to a known pharmaceutical additive, which is useful when a pharmaceutically active compound for administration is formulated and is substantially non-toxic and non-irritating under conditions of use. The exact proportion of such an excipient is determined by the solubility and chemical properties of the active compound, a selected administration route, as well as standard pharmaceutical practice.

The pharmaceutical composition of the present invention may be prepared in a form suitable for a desired administration method using additives such as appropriate and physiologically acceptable excipients, disintegrants, sweeteners, binders, coating agents, bulking agents, lubricants, glidants, and flavoring agents.

The pharmaceutical composition may be formulated in the form of a tablet, a capsule, a pill, granules, powder, an injection, a film, or a liquid, but the present invention is not limited thereto.

The dosage form of the pharmaceutical composition and the pharmaceutically acceptable carrier may be appropriately selected according to techniques known in the art, and for example, reference may be made to the following documents: [Urquhart et al., Lancet, 16:367, 1980]; [Lieberman et al., PHARMACEUTICAL DOSAGE FORMS-DISPERSE SYSTEMS, 2nd ed., vol. 3, 1998]; [Ansel et al., PHARMACEUTICAL DOSAGE FORMS & DRUG DELIVERY SYSTEMS, 7th ed., 2000]; [Martindale, THE EXTRA PHARMACOPEIA, 31st ed.]; [Remington's PHARMACEUTICAL SCIENCES, 16th-20th editions]; [THE PHARMACOLOGICAL BASIS OF THERAPEUTICS, Goodman and Gilman, eds., 9th ed., 1996]; [Wilson and Gisvolds' TEXTBOOK OF ORGANIC MEDICINAL AND PHARMACEUTICAL CHEMISTRY, Delgado and Remers, eds., 10th ed., 1998]. In addition, for principles of formulating the pharmaceutical composition, reference may be made to the following documents [Platt, Clin Lab Med, 7:289-99, 1987]; [Aulton, PHARMACEUTICS: THE SCIENCE OF DOSAGE FORM DESIGN, Churchill Livingstone, NY, 1988]; [EXTEMPORANEOUS ORAL LIQUID DOSAGE PREPARATIONS, CSHP, 1998], ["Drug Dosage," J Kans Med Soc, 70(1):30-32, 1969].

In one embodiment, the pharmaceutical composition of the present invention may be formulated as an injection, but the present invention is not limited thereto. The injection may be formulated as an intravenous or subcutaneous injection. A parenteral injection may include components included in general injection compositions. For example, the injection composition includes, for example, a liquid carrier such as sterile water, water for injection, or physiological saline. In addition, the pharmaceutical composition of the present invention may further include one or more components selected from the group consisting of amino acids, sugars, lipids, vitamins, electrolytes, pH adjusters, stabilizers, osmotic pressure adjusters, and solubilizing agents.

Currently, apomorphine has been developed as a subcutaneous injection, and is marketed under the brand name "Apokyn." Apokyn was developed to be injected subcutaneously 3 to 5 times a day, and the pharmaceutical composition of the present invention may also be formulated into the same formulation as Apokyn if necessary. For example, the pharmaceutical composition of the present invention may be a subcutaneous injection, and in this case, in addition to apomorphine, sodium metabisulfite, benzyl alcohol, hydrochloric acid, sodium hydroxide, and the like may be included as additives.

In one embodiment, the pharmaceutical composition of the present invention can be formulated as a sublingual tablet or film. When the composition is formulated as a tablet, it may further include one or more components selected from the group consisting of an excipient, a binder, a disintegrant, a lubricant, a solvent, an antioxidant, an emulsifier, a thickener, an anti-caking agent, a flavoring agent, a colorant, a sweetener, a pH adjuster, a sustained-release agent, a stabilizer, and a coating agent. In one embodiment of the present invention, the additives may include sodium edetate, ascorbic acid, magnesium stearate, hypromellose, citric acid, microcrystalline cellulose, colloidal silicon oxide, cool mint orange (WONF WL-28499), red 30 iron oxide (E172), acesulfame potassium, and mannitol, but the present invention is not limited thereto.

When the composition is formulated as a film, it may further include a binder, a stabilizer, an antioxidant, a chelating agent, a thickener, a coating agent, a flavoring agent, a sweetener, and a pH adjuster. The additives may include, but are not limited to, sodium metabisulfite, disodium EDTA, dihydrate, glycerol, glyceryl monostearate, hydroxyethylcellulose, hypromellose, maltodextrin, (-)-menthol, pyridoxine hydrochloride, sodium hydroxide, sucralose, and white ink.

A preferable dose of the pharmaceutical composition of the present invention may depend on a subject's condition and body weight, the severity of a disease, a drug type, an administration route and administration duration, and may be suitably selected by those of ordinary skill in the art. However, for desirable effects, the composition of the present invention can be administered at 100 µg to 200 mg, e.g., 200 µg to 100 mg, or 2 mg to 6 mg at a time. Administration may be performed once a day or in divided doses. Therefore, the above dose range does not limit the scope of the present invention in any way.

In the present invention, "subject" refers to a target in need of prevention or treatment of a disease, and more specifically, a mammal such as a human or a non-human primate, a mouse, a rat, a dog, a cat, a horse, or a cow in need of prevention or treatment of a necroptosis-related disease.

The composition may be administered via various routes. All modes of administration may be considered, and may be, for example, oral, transdermal, sublingual, rectal, intravenous, intramuscular, subcutaneous, intranasal, intrauterine or intracerebroventricular administration.

In one exemplary embodiment of the present invention, the pharmaceutical composition may be administered by subcutaneous injection.

The advantages and features of the present invention and the methods of accomplishing the same will become apparent with reference to the following examples to be described in detail and the accompanying drawings. However, the present invention is not limited to the exemplary embodiments disclosed below and may be embodied in many different forms. These exemplary embodiments are merely provided to complete the disclosure of the present invention and fully convey the scope of the present invention to those of ordinary skill in the art, and the present invention should be defined only by the scope of the accompanying claims.

### [Examples]

### Experimental methods

### 1-1. Cell lines, culture, and transfection

A human monocyte cell line THP-1 and a human colon cancer cell line HT-29 were maintained in a 10% FBS-Roswell Park Memorial Institute (RPMI) 1640 medium supplemented with 2 mM L-glutamine, 100 U/ml penicillin, and 100 µg/ml streptomycin at 37 °C under a 5% CO₂ condition. The THP-1 cells were differentiated into macrophages in an RPMI 1640 medium containing 500 nM Phorbol 12-myristate 13-acetate (PMA) for 3 hours. Human mixed lineage kinase domain-like protein (MLKL)-flag plasmid (Sino Biological, HG12810-CF) transfection was performed using the electroporation system (Invitrogen) of MicroPorator-mini (Digital Bio).

### 1-2. Animal experiment

An animal experiment was performed on 6 to 8-week-old male C57BL/6 mice in accordance with the procedures approved by the Animal Care and Use Committee of the Yonsei Laboratory Animal Research Center (YLARC, 2018-0024) at Yonsei University. The mice were housed in polypropylene cages with a maximum of four mice per cage under constant temperature and humidity, and the light/dark cycle was 12 hours/12 hours. All mice were given an acclimation period of at least 7 days before participation.

### 1-3. DSS-induced colitis mouse model

For colitis induced by dextran sulfate sodium (DSS, 36-50 kDa, MP Biomedicals), the mice were administered 2.5% DSS in drinking water for 7 days. The mice were randomly divided into 8 groups as follows.
1) Control
2) Apomorphine-treated group (=APO; STK088477, Vitas-M, 1.5 mg/kg, daily intraperitoneal injection)
3) APO-treated group (15 mg/kg, daily intraperitoneal injection)
4) DSS-treated group
5) DSS and necrostatine-1-treated group (NEC-1; Biovision, 1864-5, 1.5mg/kg, daily intraperitoneal injection)
6) DSS and APO-treated group (0.15mg/kg, daily intraperitoneal injection)
7) DSS and APO-treated group (1.5mg/kg, daily intraperitoneal injection)
8) DSS and APO-treated group (15mg/kg, daily intraperitoneal injection)

The body weights and disease activity index (DAI) scores of the mice were recorded daily. DAI was calculated by adding scores of body weight loss (0 point = none, 1 point = 1% - 5% weight loss, 2 points = 5% - 10% weight loss, 3 points = 10% - 15% weight loss, 4 points = 15% or more weight loss), stool consistency/diarrhea (0 point = normal, 2 points = loose stools, 4 points = watery diarrhea), and bleeding (0 point = no bleeding, 2 points = slight bleeding, 4 points = total bleeding). At the end of the experiment, the mice were sacrificed, the colon was removed, and then changes in colon length were confirmed, and a weight loss was measured.

In this experiment, NEC-1 was used as a material for inhibiting RIP1 phosphorylation and used as a control for confirming the effects of apomorphine.

### 1-4. Preparation of tissue sample

At the end of the experiment, the mice were sacrificed, and the colon was removed (without the cecum). The removed colon was washed with cold phosphate-buffered saline (PBS; Welgene, LB001-02) before the measurement of its length. The measured colon was prepared for further analysis by cutting intestinal segments along the mesenteric line and washing with PBS. Then, the intestinal segments were placed in a petri dish and fixed with modified Bouin's fixative (50% ethanol / 5% acetic acid in dH₂O). The lumen side was maintained upward by holding the proximal end with forceps in one hand and a toothpick in the other. Then, the flat open end of the colon was pulled with the forceps and the proximal end was wrapped around the toothpick. The entire large intestine was wrapped, placed in a cassette, and immersed in 4% paraformaldehyde (Biosesang, PC2031-100-00) at 4 °C overnight.

### 1-5. Necroptosis induction

Cells were pretreated with Z-VAD (invivoGen, tlrl-vad) and a compound for 1 hour, and necroptosis was induced with TNF-α (ATGen, TNF0501) and BV6 (Biovision, B1332-5) for the indicated time. The prepared cells were appropriately treated according to analysis (Z-VAD : carbobenzoxy-valyl-alanyl-aspartyl, TNF-α : tumor necrosis factor-α, BV6 : 4,4'-(1,6-hexanediyl)bis[N-methyl-L-alanyl-(2S)-2-cyclohexylglycyl-L-prolyl-β-phenyl-L-phenylalaninamide)

### 1-6. Western blot analysis

Cells were treated with a drug candidate and then collected using centrifugation at 4 °C and 1,500 xg for 5 minutes. A supernatant was centrifuged again at 4 °C and 1,500 xg for 5 minutes to remove remaining debris, concentrated at 4 °C and 14,000 xg for 30 minutes using Amicon ultra-0.5 centrifugal filter unit 10K (Millipore, UFC501096), and stored. The collected cells were lysed using 1x radioimmunoprecipitation assay (RIPA) buffer (GenDEPOT, R4100-010) containing a 1X protease inhibitor (GenDEPOT, P3100-001) and a phosphatase inhibitor (Thermo Fisher Scientific, 1862495). Subsequently, the cell lysate was centrifuged at 4 °C and 20,000 xg for 30 minutes to remove debris. The extracted protein concentration was measured using a bicinchoninic acid (BCA) protein assay kit (Thermo Fisher Scientific, 23225). The supernatant that had been concentrated and stored and the extracted protein were subjected to non-reducing or reducing SDS-PAGE (8%-12%), and the SDS-PAGE gel was transferred to a nitrocellulose membrane. The membrane was blocked with 5% nonfat milk in Tris buffered saline-1% Tween 20 (TBS-T), and investigated with antibodies specific for MLKL (Cell Signaling Technology, 14993S), phospho-MLKL (S358) (Cell Signaling Technology, 91689S), GAPDH (Abfrontier, LF-PA0018), phspho-RIP1 (S166) (Cell Signaling Technology, 65746S), RIP1 (Abcam, ab72139), LDH (Cell Signaling Technology, 2012S), and LAMP1 (Abcam, ab24170), HMGB1 (Abcam, ab92310).

### 1-7. Subcellular fractionation

Collected cells were resuspended in 20 mM Tris (pH 7.4) buffer supplemented with 10 mM KCl, 1 mM MgCl₂, a 1X protease inhibitor, and a phosphatase inhibitor and incubated on ice for 30 minutes. The cells were passed through a 22-gauge needle 30 times and centrifuged at 500 xg for 15 minutes. The supernatant was centrifuged again at 20,000 xg for 15 minutes, and stored as a cytoplasmic fraction. The remaining pellet was resuspended with lysis buffer and centrifuged at 20,000 xg for 15 minutes, and stored as a crude membrane fraction.

### 1-8. Microplate reader for measuring propidium iodide (PI) uptake in pore-forming cells

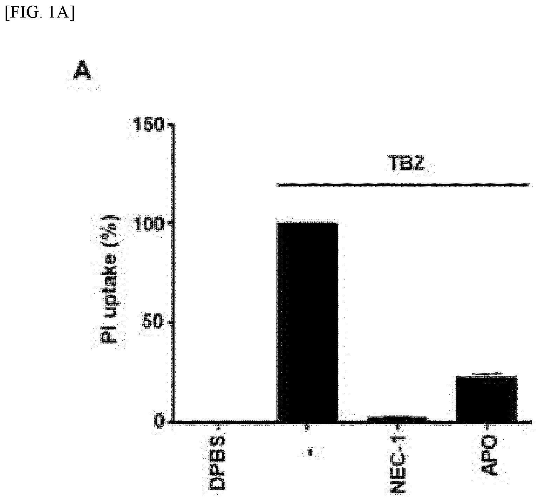

Cells were plated in a flat, transparent-bottomed, black-walled 96-well plate, and subjected to 7-hour chemical treatment and necroptosis induction, and then PI (BD Biosciences, 556463) was added to a final concentration of 50 µg/ml. After incubation for 10 minutes under the original culture conditions, the cells were centrifuged at 400 xg for\ 5 minutes. The emission wavelength at 617 nm following excitation at 530 nm was measured by real-time fluorescence monitoring on a Varioskan Flash 3001 plate reader. Data collection was performed at 37 °C (FIG. 1A).

### 1-9. Immunofluorescent staining

Cells were seeded on cell culture-treated glass chamber slides. The cells were pretreated with Z-VAD, BV6, and the indicated compound for 1 hour, and then treated with TNF-α and BV6 overnight. Subsequently, the cells were washed with phosphate-buffered saline (PBS) and fixed in 4% paraformaldehyde for 10 minutes. Before incubation for 10 minutes in 0.25% Triton X-100 in PBS, the cells were washed three more times with PBS. The cells were blocked with 5% bovine serum albumin (BSA) in PBS for 30 minutes and stained using an anti-Flag (Sigma, F7425-2MG) antibody. The nuclei were stained with DAPI (Invitrogen, 00-4959-52). Images were captured using a confocal microscope (Olympus, FV1000).

### 1-10. Surface plasmon resonance (SPR) analysis

The interaction between MLKL and apomorphine (=APO) was detected through surface plasmon resonance (SPR) analysis using Biacore T200 (GE Healthcare). For pH scouting, a buffer consisting of 10 mM sodium acetate (pH 4.0 to 6.0) was used. MLKL protein (Abcam, ab241453), and MLKL N-term (amino acids 1-154) protein that had been purified in a laboratory were immobilized on the surface of a sensor chip CM5 (GE Healthcare) under scouted buffer conditions. To deduce an R_{ligand} value, a coupling process was performed based on the molecular weights of MLKL, MLKL N-term domain (MW ligand), and APO (MW analyte). Oxidized (24 hr, RT incubation) and reduced (+DTT(DL-dithiothreitol)) forms of APO were injected into a running buffer (flow rate: 10 µl/min) consisting of 10 mM HEPES (pH 7.4) at 25 °C. A sensorgram was recorded and analyzed in real time using control software provided by the Biacore T200 system itself. Six or more concentrations of APO were used in combination, and each K_{D} value was derived. All run data was calculated using BIAevaluation Software (GE Healthcare).

### 1-11. Histological evaluation

After the experiment, colon tissues collected from the sacrificed mice were fixed in 4% paraformaldehyde overnight. Swiss-rolled and fixed colon specimens were embedded in paraffin. The specimens were cut into 5-µm sections, and then the sections were stained with hematoxylin and eosin (H & E). The histological evaluation of colitis lesions along the entire length was performed with reference to a scoring system of the degree of crypt structure damage (0-3), the degree of inflammatory cell infiltration (0-3), and submucosal edema (0-3).

### 1-12. Immunohistochemistry (IHC) and periodic acid-Schiff (PAS)

5-µm cut paraffin sections were blocked with 5% BSA-supplemented TBS-T, and then the sections and primary antibodies were co-incubated at 4 °C overnight. Secondary antibodies were added and incubated for 1 hour. The resulting sections were incubated together with a 3,3'-diaminobenzidine (DAB) kit for 10 minutes. Periodic acid staining (PAS) staining was performed according to the standard protocol.

### 1-13. Statistical analysis

The analysis of experimental data was performed using GraphPad Prism. Data represents the mean value, SD, and SEM as indicated in individual figure legends. Differences were considered statistically significant when * p < 0.05, ** p < 0.01, and *** p < 0.001.

### Experimental Example 1: Confirmation of cell membrane pore-forming ability upon apomorphine treatment

As a first screening process, to investigate changes in cell membrane integrity by measuring PI uptake (Experimental Method 1-8), a compound in which the PI uptake had been reduced to less than 30% of the signal intensity upon TBZ treatment was selected.

After treating TBZ+APO-treated THP-1 cells with PI for 10 minutes, the PI taken up by the cells was measured on a microplate reader. As an inhibitor control, necrostatin-1 (NEC-1) was used. Data is presented as the average of two experiments (TBZ : TNF- α + BV6 + Z-VAD).

As a result, apomorphine (APO) effectively reduced a PI uptake rate, and reduced the ability of the selected compound to reduce cell membrane pore formation (FIG. 1A).

### Experimental Example 2: Confirmation of MLKL phosphorylation inhibition effect of apomorphine

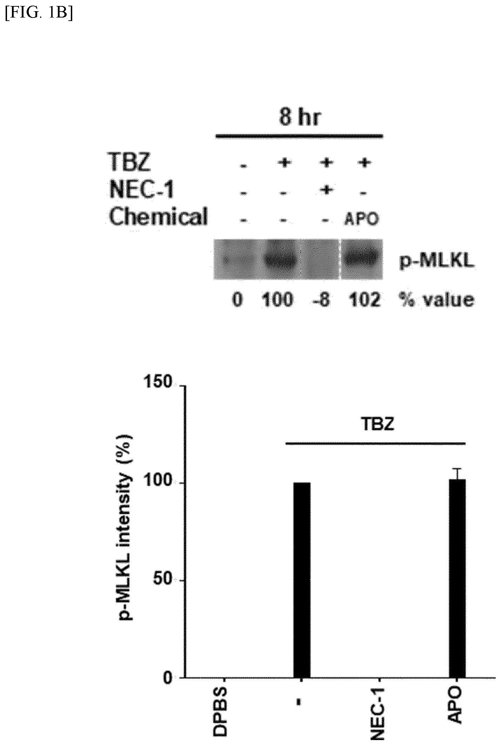

As a second screening process, Western blotting was performed for the functional analysis of MLKL phosphorylation (Experimental Method 1-6, FIG. 1B). Western blotting using the indicated antibodies shows changes in MLKL phosphorylation in TBZ+APO-treated cells. Data is presented as the average of two independent experiments.

As a result, APO did not inhibit MLKL phosphorylation. That is, as confirmed in Experimental Example 1, APO reduces cell membrane pore formation, indicating that APO affects the process from MLKL phosphorylation to cell membrane pore formation to inhibit necroptosis.

### Experimental Example 3: Confirmation of apomorphine-related necroptosis pathway

To confirm which step of the necroptosis pathway is affected by apomorphine, TBZ, NEC-1, and APO-treated cells were fractionated into cytoplasmic and crude membrane fractions, and then samples were subjected to Western blotting with the indicated antibodies.

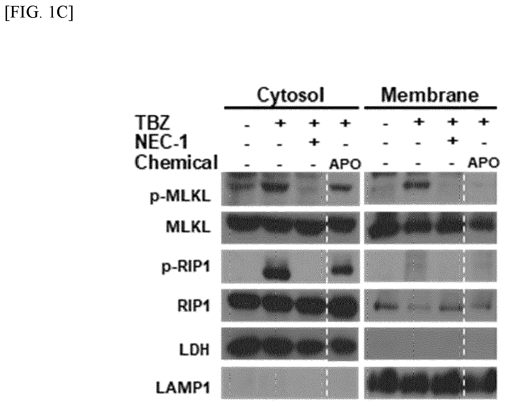

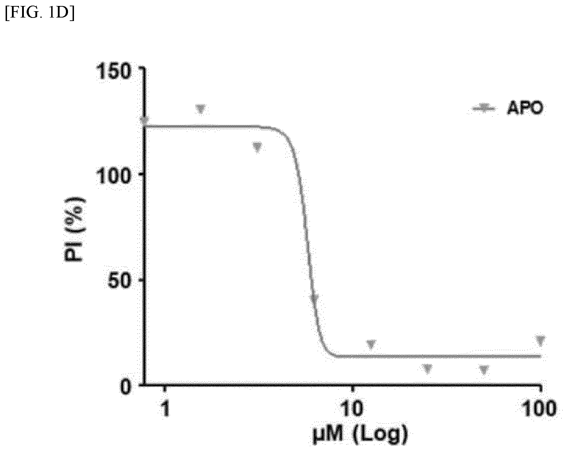

As a result, through the fractionation of the cell cytoplasm and membrane, the phosphorylation of RIP1 and MLKL, which are mediators of the necroptosis pathway, and the translocation of the molecules from the cytoplasm to the membrane fraction were confirmed (FIG. 1C). In addition, the inhibitory concentration (IC50) of a drug causing 50% of maximum inhibition was confirmed by measuring the change in cell membrane integrity in a concentration-dependent manner for APO (IC50=5.7 µm) (FIG. 1D).

### Experimental Example 4: Confirmation of inhibition of MLKL oligomerization by apomorphine

To test whether the tetramer and octamer formation of p-MI,KL for pore formation in the cell membrane is inhibited by apomorphine (APO), cells that had been treated with each molecule for the indicated time were separated into cytoplasmic and membrane fractions and analyzed through non-reducing or reducing sodium dodecyl sulfate-polyacrylamide gel electrophoresis (SDS-PAGE), and then investigated with specific antibodies (Experimental Method 1-6). Here, lactate dehydrogenase (LDH) is a cytoplasmic marker, and lysosomal-associated membrane protein 1 (LAMP1) is a membrane marker.

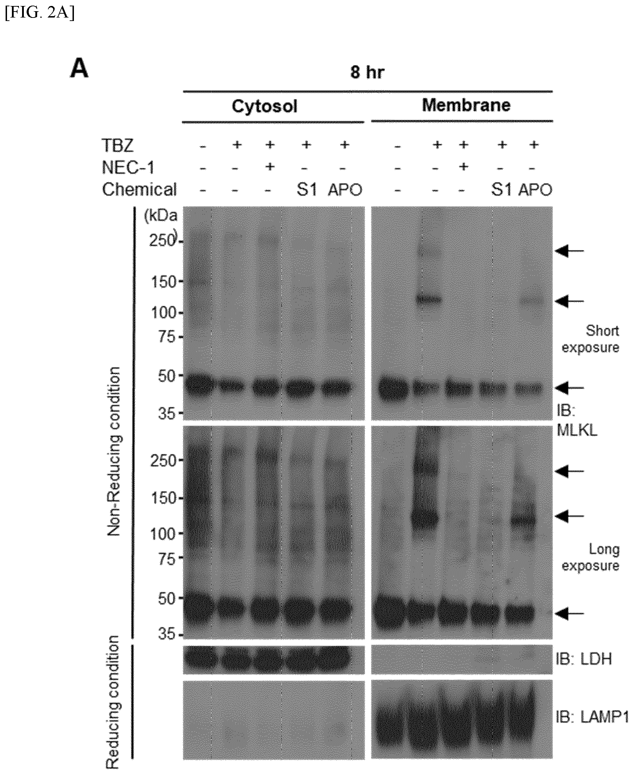

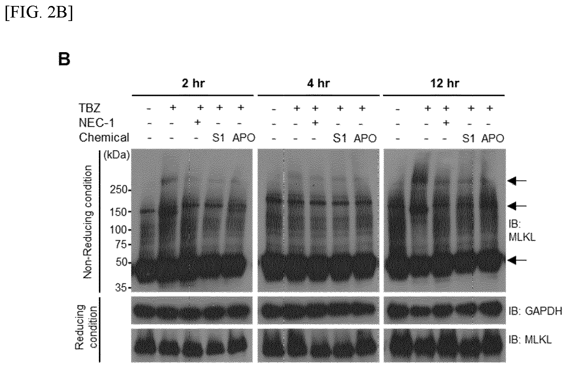

As a result, when APO was treated, it was confirmed that the intensities of the tetramer and octamer bands of MLKL decreased (FIG. 2). That is, APO inhibits MLKL oligomerization. Particularly, in FIG. 2B, after necroptosis induction, MLKL oligomerization was confirmed over time.

### Experimental Example 5: Confirmation of blocking MLKL translocation to cell membrane by apomorphine

Transfected THP-1 cells were treated with TBZ or NEC-1, and APO, and stained with DAPI and Alexa 488. The intracellular location of MLKL in the transfected THP-1 cells was tracked through the expression of MLKL-flag protein using a confocal microscope (Experimental Method 1-9).

Cells treated with TBZ to induce necroptosis showed MLKL phosphorylation and p-MI,KL translocation to the plasma membrane. While the Flag-MLKL signal was distributed throughout the cells under physiological conditions, the TBZ-treated cells showed a distribution of dot like Flag-MLKL signals in the cell membrane.

The Flag-MLKL signals were mostly distributed in the cytoplasm of the cells stimulated by APO and TBZ, similar to those stimulated by NEC-1, which is an RIP1 phosphorylation inhibitor, and TBZ.

Through this, it can be confirmed that APO inhibits the concentrated distribution of MLKL to the cell membrane in the cytoplasm.

### Experimental Example 6: Confirmation of inhibition of HMGB1 secretion by concentration-dependent treatment with apomorphine

To test whether secretion of HMGB1, a representative damage-associated molecular pattern, which is one of the characteristics of necroptosis, is inhibited by treating THP-1 cells with necroptosis induced by TBZ treatment with APO, after concentration-dependent treatment (1, 5, 20, 80, or 320 µM) with APO for 8 hours, all of the supernatant was concentrated, analyzed by SDS-PAGE, and investigated with an HMGB1 antibody (Experimental Method 1-6). As an inhibitor control, necrostatin-1 (NEC-1) was used, and data is presented as an average of three independent experiments.

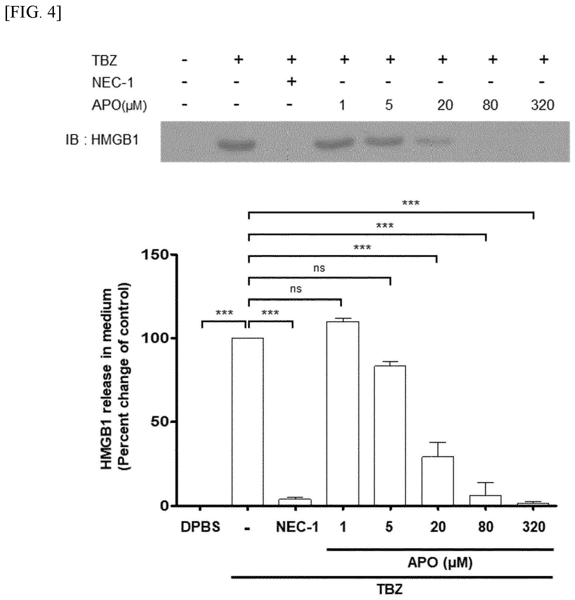

As a result, based on the TBZ only-treated group, it was confirmed that the amount of HMGB 1 secreted into the supernatant is decreased as APO is treated in a concentration-dependent manner (FIG. 4). To this end, it can be confirmed that necroptosis is inhibited by APO treatment by reducing HMGB1 secreted through the necroptosis process.

### Experimental Example 7: Confirmation of inhibition of necroptosis pathway by MLKL binding with apomorphine

Through the above experimental examples, it was confirmed that APO inhibits p-MI,KL oligomerization, thereby inhibiting cell membrane pore formation. In this example, it was confirmed whether inhibition is achieved through direct binding of APO to MLKL, especially to the MLKL N-term domain.

SPR analysis was used to analyze the interaction pattern between APO and the MLKL protein (Experimental Method 1-10). A K_{D} value of 1.272E-8M was obtained by the binding of MLKL and APO, and a K_{D} value of 6.254E-8M was obtained by the binding of MLKL N-term and APO. Here, the analysis was performed by binding MLKL and the MLKL N-term domain protein immobilized on a CM5 chip with APO having six concentrations.

That is, when APO is treated, it can be confirmed that it is directly bound to the N-term domain of MLKL with high affinity, thereby inhibiting MLKL oligomerization.

### Experimental Example 8: Confirmation of inhibition of MLKL oligomerization according to APO oxidation

It is already well known that APO is oxidized spontaneously over time in the presence of light and air. In this example, to test whether MLKL oligomerization is decreased by increased binding of APO with MLKL in cells depending on the degree of oxidation, APO was stored at room temperature for 24 hours to prepare its oxidized form, and APO dissolved immediately before the experiment was treated with DTT to prepare its reduced form. The cells treated with each molecule were lysed for the indicated time, analyzed through non-reducing or reducing SDS-PAGE, and then investigated with specific antibodies (Experimental Method 1-6).

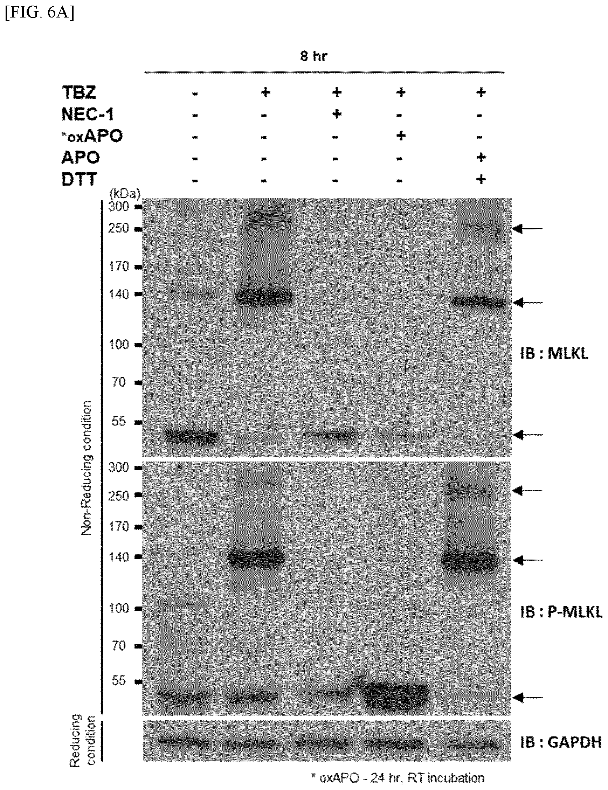

As a result, it can be confirmed that when the oxidized APO was treated, the intensities of MLKL tetramer and octamer bands were reduced, and when the reduced APO was treated, there was no change (FIG. 6A). Through this, it was confirmed that MLKL oligomerization was inhibited according to the oxidation of APO, and to confirm that such inhibition occurred as the binding of APO with MLKL increased depending on the degree of APO oxidation, the interaction pattern between MLKL and APO according to oxidation was analyzed through SPR (Experimental Method 1-10).

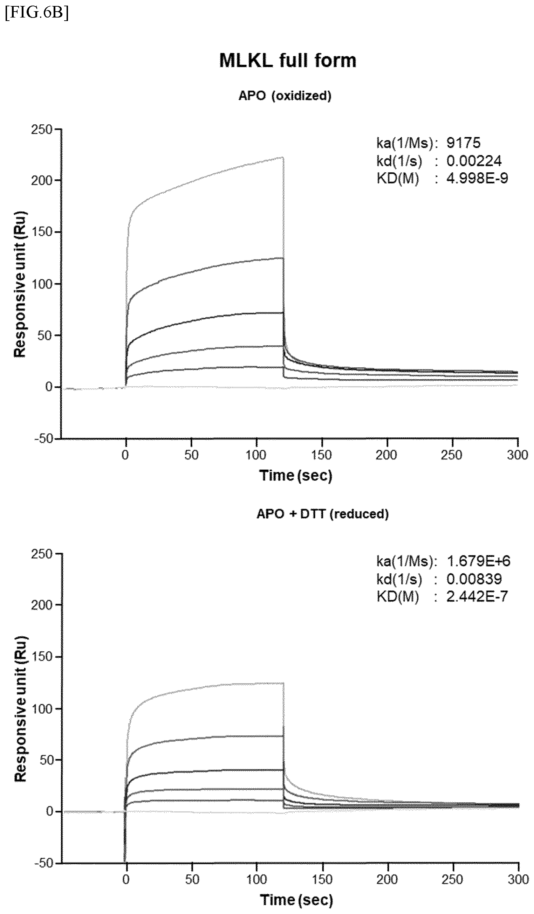

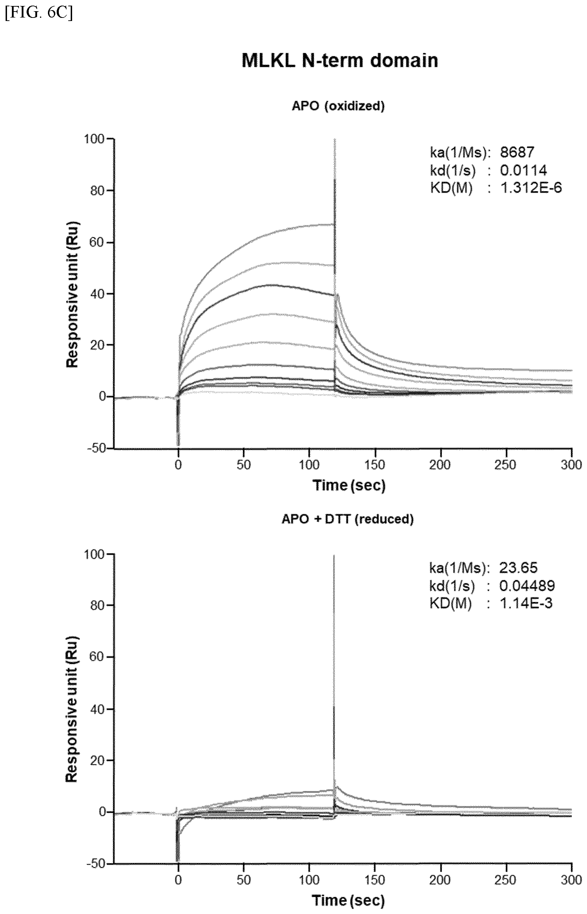

As a result, a K_{D} value of 4.998E-9M was obtained by the binding of oxidized APO with the MLKL protein and a K_{D} value of 2.442E-7M was obtained by the binding of reduced APO with the MLKL protein (FIG. 6B), and a K_{D} value of 1.312E-6M was obtained by the binding of oxidized APO with the MLKL N-term domain and a K_{D} value of 1.14E-3M was obtained by the binding of the reduced APO with the MLKL N-term domain (FIG. 6C). Here, the analysis was performed by binding MLKL and the MLKL N-term domain protein immobilized on a CM5 chip with APO having six or more concentrations.

That is, it was confirmed that, as APO oxidation occurs, the direct binding of APO with MLKL increases, thereby inhibiting MLKL oligomerization.

### Experimental Example 9: Confirmation of improvement of DSSinduced colitis by apomorphine

To confirm *in vivo APO* effects, DSS-induced colitis mouse models that mimic the symptoms of human colitis were prepared. The alleviation of colitis symptoms in mice was measured daily by body weight and a DAI score, and colon lengths, H&E, PAS staining, immune responses, and mucosal barrier damage of the mice were measured and then compared.

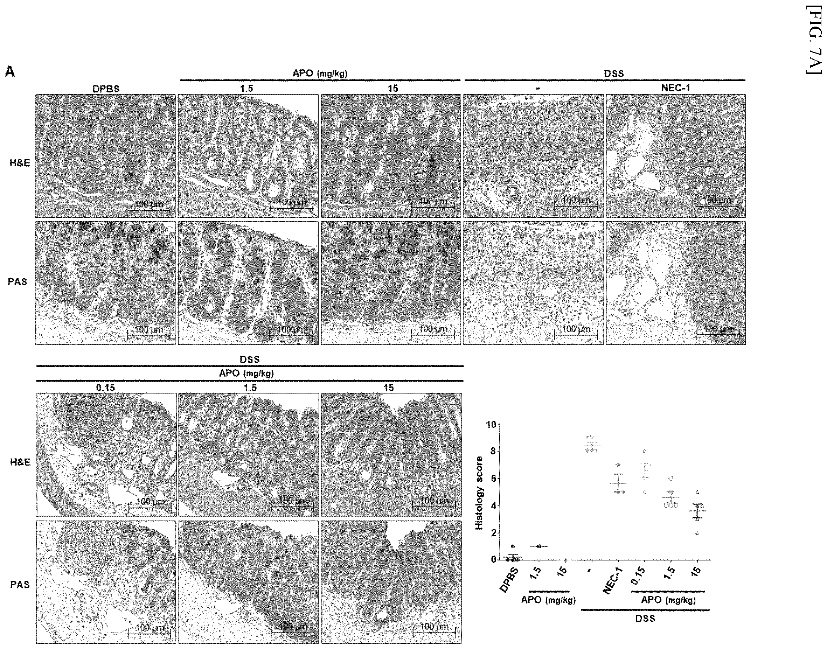

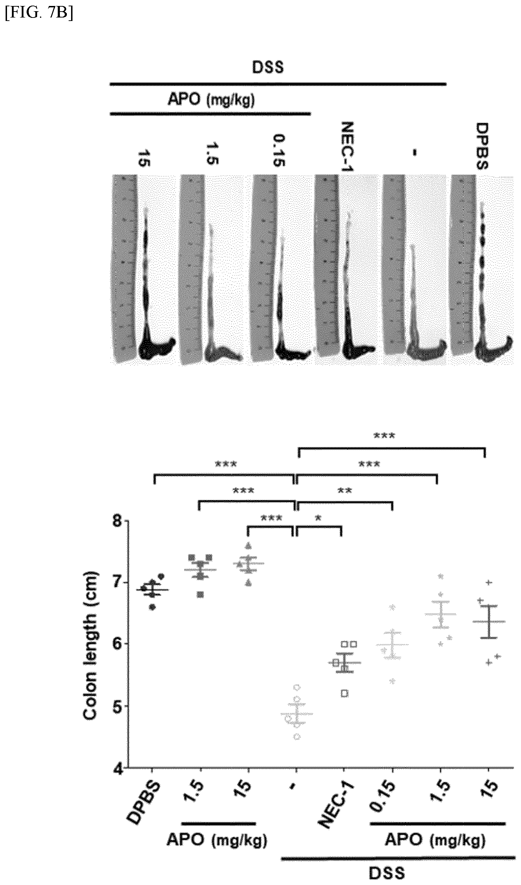

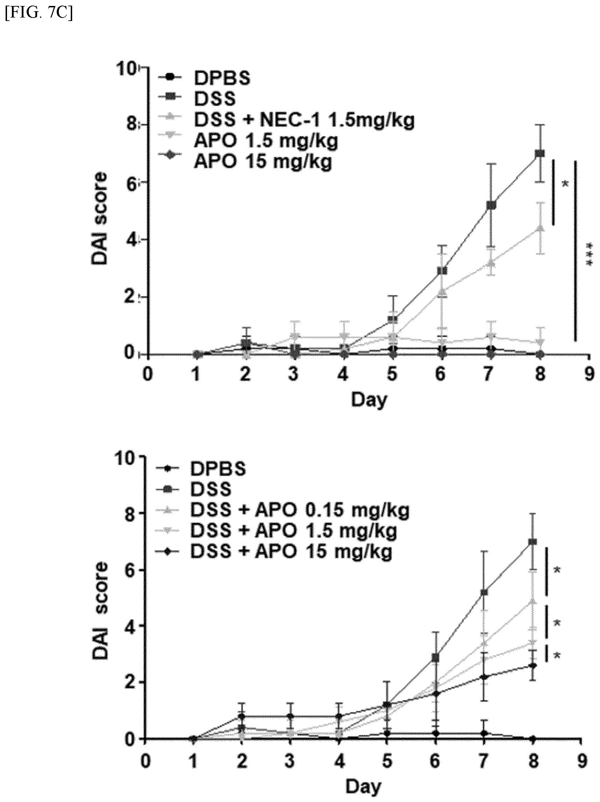

As shown in FIG. 7A, the mice in the DSS group exhibited damage to the crypt structure, inflammatory cell infiltration, and submucosal edema due to colitis. A mucus material was also significantly reduced in PAS staining, indicating that the secretion of colonic mucus generally observed in IBD patients was significantly reduced. In the groups injected with 0.15, 1.5, and 15 mg/kg of APO, the characteristic symptoms of colitis that can be observed by H&E staining and PAS staining were alleviated in a concentration-dependent manner. APO treatment also appeared to reduce an HI score (FIG. 7A), the degree of colon shortening (FIG. 7B), and a DAI score (FIG. 7C).

In summary, there were no differences due to treatment with APO alone, and the harmful effects of DSS treatment were improved by the concentration-dependent treatment of APO. That is, these results indicate that APO has a protective effect against DSS-induced colitis.

## Claims

1. A pharmaceutical composition for use in preventing or treating a necroptosis-related disease, comprising:
apomorphine or a pharmaceutically acceptable salt thereof; and
a pharmaceutically acceptable carrier.

2. The pharmaceutical composition for use of claim 1, wherein apomorphine binds with mixed lineage kinase domain-like pseudokinase (MLKL) to inhibit oligomerization.

3. The pharmaceutical composition for use of claim 1, wherein the necroptosis-related disease includes stroke, autoimmune diseases, inflammatory bowel disease (IBD), sepsis, chronic obstructive pulmonary disease, acute respiratory distress disorder, transfusion-related acute lung injury, transplant rejection, atherosclerosis, aortic aneurysms, myocardial infarction, terminal ileitis, acute kidney injury, renal ischemia reperfusion injury, liver injury, steatohepatitis, pancreatitis, and bone marrow failure.

4. The pharmaceutical composition for use of claim 3, wherein the necroptosis-related disease is inflammatory bowel disease.

5. The pharmaceutical composition for use of claim 4, wherein the inflammatory bowel disease is Crohn's disease or ulcerative colitis.

6. The pharmaceutical composition for use of claim 1, wherein the pharmaceutically acceptable salt is selected from the group consisting of an inorganic acid salt, an organic acid salt, an amino acid salt, a sulfonic acid salt, a metal salt, and an ammonium ion salt.

7. The pharmaceutical composition for use of claim 1, wherein the pharmaceutical composition includes apomorphine hydrochloride.

8. The pharmaceutical composition for use of claim 1, wherein the pharmaceutical composition is an injection.

9. The pharmaceutical composition for use of claim 7, wherein the injection includes one or more selected from the group consisting of sodium metabisulfite, benzyl alcohol, hydrochloric acid, and sodium hydroxide.
